# EUROPEAN PATENT SPECIFICATION

(11) **EP 0 461 174 B1**
(45) Date of publication and mention of the grant of the patent: **17.11.1994**
(21) Application number: 90904448.9
(22) Date of filing: 02.03.1990
(51) Int. Cl.: G01N 33/68, G01N 33/573, C12P 21/00

(54) **METHOD AND KIT FOR DETERMINING TYROSINE-PHOSPHORYLATING AND DEPHOSPHORYLATING ACTIVITIES**
VERFAHREN UND TESTSATZ ZUR BESTIMMUNG VON TYROSINPHOSPHORYLIERUNGS- UND DEPHOSPHORYLIERUNGSAKTIVITÄTEN
PROCEDE ET KIT DETERMINANT LA PHOSPHORYLATION DE TYROSINE AINSI QUE DES ACTIVITES DE DEPHOSPHORYLATION

(30) Priority: 03.03.1989 NL 8900529
(43) Date of publication of application: 18.12.1991
(73) Proprietor: FERRING N.V., Willemstad, Curaçao (AN)
(72) Inventor: RIJKSEN, Gerrit, NL-3732 GL De Bilt (NL)
(74) Representative: de Bruijn, Leendert C.
(86) International application number: NL9000023
(87) International publication number: WO9010234

(56) References cited:
- US-A- 4 543 439
- BIOLOGICAL ABSTRACTS, vol. 88, no. 12, 1989; G. RIJKSEN et al., p. 317, no. 129533#
- JOURNAL OF IMMUNOLOGICAL METHODS, vol. 109, no. 2, 1988, Elsevier Science Publishers B.V., Amsterdam (NL); J.R. GLENNEY et al., pp. 277-285#

## Description

The invention relates to a method for determining the tyrosine-phosphorylating or phosphotyrosine-dephosphorylating activity of a material to be investigated, such as an enzyme, by bringing the material into contact with a polypeptide containing tyrosine or phosphotyrosine respectively and a phosphate source or phosphate acceptor respectively and then assaying the phosphorylated polypeptide.

The determination of the tyrosine-phosphorylating or phosphotyrosine-dephosphorylating activity of material, in particular of biological material, is of great importance for diagnostic and prognostic purposes and, in addition, for research purposes. Thus, it is known that the phosphorylation of tyrosine is an important response mechanism to a number of biological processes such as the action of growth factors and oncogenes [Y. Yarden, Ann. Rev. Biochem. 57, 443-478 (1988); T. Hunter and J.A. Cooper in The Enzymes (edited by P.D. Boyer and E.G. Krebs), vol. XVII, pages 191-246, Acad. Press, Orlando, Fl. (1986)]. In breast cancer cells and also in other solid tumours, for example, the expression of oncogenes and growth factor receptors appears to result in a considerably increased tyrosine kinase activity, while in benign tumours, a less markedly increased activity is observed [H. Hennipman et al., Cancer Research, 49, 516-521 (1989)]. In the case of leukaemia reduced tyrosine phosphatase activity, i.e. a reduced phosphotyrosine dephosphorylation, is reported [D.A. Frank and A.C. Sartorelli, Cancer Res. 48, 52-58 (1988)]. In experimental systems it has also been shown that the addition of a specific tyrosine phosphatase inhibitor can give rise to a malign degeneration of cells [J.K. Klarlund, Cell 41, 707-717 (1985)].

To detect deviations in the cell growth and to determine the nature thereof, there is therefore a need for reliable and efficient methods for determining the tyrosine kinase activity or tyrosine phosphatase activity

The determination of the tyrosine kinase activity of breast tissue fractions by reacting the sample with a tyrosine-containing polypeptide and adenosine triphosphate labelled with radioactive phosphorus (^{32p}-ATP), after which the radioactive label incorporated in the polypeptide is measured, is known [A. Hennipman et al., Cancer Research, 49, 516-521 (1989)].

This method has the disadvantages which are always associated with a radioactive assay, viz. that the latter is cumbersome, requires safety precautions and cannot easily be carried out in large series. In addition, the use of radioactively labelled ATP has the further drawback that the ATP concentration in the determination medium may not be optimally chosen. Furthermore, an appreciable background signal is found, which is formed by the phosphorylation of other substrates present in the sample and irrelevant to the determination such as serine residues in proteins, and this results in a variable and sometimes inadequate limit of detection.

A method has now been found which does not have the disadvantages and drawbacks mentioned. This method can be carried out simply and without safety risks, it shows considerably less noise and background signals and thus needs little if any corresponding correction, and it has one and the same sensitivity and activity as, or better sensitivity and activity than, the known method.

According to the method of the invention for determining the tyrosine-phosphorylating or phosphotyrosine-dephosphorylating activity of a biological material, the material is brought into contact with a polypeptide containing tyrosine or phosphotyrosine respectively and a radioactively unlabelled phosphate source or phosphate acceptor respectively and the phosphorylated polypeptide is then assayed by using an antibody to polypeptide containing phosphotyrosine.

The antibodies against the polypeptide containing phosphotyrosine to be used in the method according to the invention may be polyclonal or monoclonal. Monoclonal and polyclonal phosphotyrosine antibodies have been described (see J.Y.J. Wang, Anal. Biochem. 172, 1-7 (1988) for a review article). The use of polyclonal antibodies has, as is known, the drawback of a low specificity and reproducibility. It is possible, however, to purify polyclonal phosphotyrosine antibodies, for example with the aid of chromatography on a tyramine column, so that a specific antibody is obtained which approaches the properties of a monoclonal antibody in its operation. The antibody to be used is preferably monospecific. Monoclonal antibodies, for example propagated in mice, against phosphotyrosine residues in proteins have been described, for example 1G2 [Huhn et al., Proc. Natl. Acad. Sci. USA, 84, 4408-4412 (1987)], and such antibodies are obtainable commercially. At least five different types of immunogens have hitherto been used to induce monoclonal or polyclonal antibodies. Three of these types are analogues of phosphotyrosine coupled to carrier proteins, viz. p-azobenzyl phosphonate, bromoacetyl-O-phosphotyramine and phosphotyrosine itself. A fourth type of immunogen is composed of an artificial polypeptide containing phosphotyrosine and the fifth type is formed by naturally occurring proteins phosphorylated to tyrosine residues. The resulting antibodies are purified by, for example, immunoaffinity chromatography on immobilized phosphotyramine and are in all cases comparable as regards activity and specificity [J.Y.J. Wang, Anal. Biochem. 172, 1-7 (1988)].

The method for carrying out an immune reaction such as that between a protein containing phosphotyrosine and an antibody is known per se [J.Y.J. Wang, Anal. Biochem. 172, 1-7 (1988)]. The immune complex can be determined for example when the antibody is provided with a label such as a radioactive isotope (RIA), for example ¹²⁵I, an enzyme (ELISA), for example horseradish peroxidase, a luminescent substance, a gold particle, biotin and the like. Another preferred method for detecting the immune complex is to provide the immunogen, i.e. the polypeptide containing tyrosine residues, with a label such as mentioned above, preferably an enzyme, instead of labelling the antibody. On the other hand, the immune complex can be determined with the aid of a second antibody against the first antibody. In the case where the first antibody is a mouse antiphosphotyrosine, such a second antibody may be, for example, an anti-mouse antibody produced in goats. Said second antibody is then preferably provided with a similar label.

As already stated, all the known labelling substances, such as enzyme labels, biotin, protein A with radioactive label, etc. are in principle suitable as a label. It has been found that an antibody labelled with gold can advantageously be used in the method according to the invention because the detection thereof has a high sensitivity. A further increase in the sensitivity can be achieved by using enhancement of the signal with silver. The use of immunogold determinations and of the silver enhancement have been described by M. Moeremans et al., J. Immunol. Meth. 74, 353-360 (1984). Suitable gold-labelled antibodies are obtainable commercially, as are the silver reagents suitable for enhancement. The (silver)gold signal can be quantified by means of densitometry.

The method according to the invention can advantageously be used to determine the phosphorylating activity of tyrosine kinases, in particular within the framework of research and of the diagnosis of deviations in the cell growth, for example in tumours. In particular, this relates to determination of the activity of protein tyrosine kinases (PTK), i.e. enzymes which catalyse the phosphorylation of tyrosine units in protein substrates.

To phosphorylate tyrosine, a phosphate source, usually adenosine triphosphate (ATP) or guanosine triphosphate (GTP), is necessary. These phosphates, and in particular ATP, are also preferably used in the method according to the invention. The quantity and the concentration of ATP can be adapted to the practical requirements and may be much higher (for example 1 mM) than is achievable in the known determination with ³²P-ATP.

The substrate for the phosphorylation reaction may be any polypeptide which contains tyrosine units and against which an antibody can be induced in phosphorylated form. This may be a natural or a synthetic polypeptide. The polypeptide also preferably contains amino acid residues other than tyrosine, for example glutamic acid, aspartic acid, alanine etc. It has been found that a polypeptide which has a random amino acid sequence and, in addition to tyrosine residues, also contains glutamic acid residues is readily phosphorylated and subsequently complexed (recognized) by an antibody. A readily usable polypeptide is, for example, a poly-GluNa/Tyr (4:1) (PGT) with a random structure.

The method according to the invention can also be used to determine the dephosphorylating activity of tyrosine phosphatases, which activity can also be related to the occurrence of irregularities in the cell growth, for example in the case of leukaemia. The phosphate acceptor. for the dephosphorylation of phosphotyrosine may, for example, be adenosine diphosphate (ADP) or guanosine diphosphate (GDP), but also water or other substances normally present in the determination medium. In keeping with the situation in the case of the phosphorylation, the substrate for the phosphatase reaction may be any polypeptide containing phosphotyrosine against which an antibody can be induced. For this purpose, a polypeptide which contains tyrosine usable for the kinase determination and of which at least some of the tyrosine residues are phosphorylated can be used, for example a synthetic polypeptide containing glutamic acid and phosphotyrosine.

The kinase or phosphatase determination can be carried out in a standard medium and under conditions which are standard for such assays. The phosphorylation reaction can be started by adding ATP to the mixture of sample, substrate and medium constituents. The reaction can be terminated by adding inhibitors such as ethylenediaminetetraacetic acid (EDTA) and/or ethyleneglycol bis(2-aminoethyl ether) N,N,N′,N′-tetraacetic acid (ECTA).

The phosphorylated polypeptide can then be attached to a solid carrier such as a membrane and can be brought into contact with the antibody, after which the immune complex is determined qualitatively or quantitatively. Instead, the polypeptide substrate can be attached to a solid carrier before the enzyme reaction is carried out, after which the further method is followed as described above. The mixture containing the phosphorylated polypeptide can also be brought into contact with an immobilized antibody and then be treated with a second antibody and be assayed. As mentioned before, it is also possible to label the polypeptide instead of the antibody, and to allow it to react with the immobilized antibody.

To carry out the method according to the invention, use can also be made of a polypeptide incorporating an additional antigen sequence. Another anchorage possibility for the immune complex is then obtained by using an additional antibody against said additional antigen.

Prior to the determination of the sample to be investigated, a calibration line can be plotted of results obtained with enzymes having known activity or of a standard of phototyrosine-containing polypeptide of which the phosphotyrosine content is known.

The invention also relates to combinations of reagents ("kits") which are suitable for use in the method described above. The kit according to the invention contains at least one tyrosine-containing polypeptide and/or a phosphotyrosine-containing peptide, a radioactively unlabelled phosphate source and/or phosphate acceptor, and an antibody to polypeptide containing phosphotyrosine. Said antibody is preferably monospecific and is, for example, monoclonal mouse antiphosphotyrosine. The antibody may be labelled, for example with a gold label. The kit may also contain a second antibody against the first antibody and possibly further agents for measuring the immune complex, such as silver as enhancer of the gold label and the like.

Furthermore, the kit may advantageously contain a buffered medium in which the determination can be carried out and also an inhibitor for the (de)phosphorylation reaction. In addition, the kit may contain a carrier for immobilizing polypeptide, for example a polyvinylidene fluoride membrane.

The kit is preferably suitable for determining kinase activity and then contains a tyrosine-containing polypeptide and also a phosphate donor such as ATP. The kit may also be directed at the determination of phosphatase activity and then comprises a phosphotyrosine-containing polypeptide.

Preferably, the kit also contains an instruction for carrying out the method described above.

### EXAMPLE I

### Materials:

Polyvinylidine difluoride (PVDF) transfer membranes (Immobilon) were purchased from Millipore (Bedford, MA, USA); bovine serum albumin (BSA) and polyglutamate-tyrosine (PGT) having a mean molecular weight of 28,500 were obtained from Sigma (St. Louis, MO, USA). [γ-³²P]ATP (10-40 Ci/mmol) originated from New England Nuclear (Berkeley, CA, USA) and unlabelled ATP from Boehringer (Mannheim, FRG). The reagents for the immunogold staining method with silver enhancement originated from Janssen (Beerse, Belgium). Monoclonal antibodies against phosphotyrosine residues (monoclonal 1G2) originated from Oncogene Science (Manhasset, NY, USA). All the other reagents were of analytical purity.

### Sources of protein tyrosine kinase (PTK) activity:

Cytosolic extracts of normal human breast tissue, benign breast tumours and malignant breast carcinomas were used as a source of PTK activity. Breast tissue samples from patients with juvenile hypertrophy were obtained while carrying out a reduction mammoplasty. Upon histological examination, these tissues did not exhibit any abnormality and were therefore regarded as normal breast tissue. Biopsies obtained after surgery were immediately brought to -80° C and stored until used. Cytosol extracts were prepared by homogenizing a tumour sample in 10 mM Tris/HCl, pH 7.4, containing 0.25 M sucrose, 1 mM MgCl₂, 1 mM EDTA, 1 mM diisopropyl fluorophosphate, 1 mM phenylmethanesulfonyl fluoride, 1 mM dithiothreitol and 0.5 mg/ml aprotinin. Debris and nuclei were removed by centrifugation at 800 g for 10 min. at 4° C. The cytosol was isolated by ultracentrifugation (150,000 g, 30 min., 4° C) and used as a source of PTK activity in the assays.

### Determination of PTK activity by means of the filter paper assay with trichloroacetic acid (TCA):

The tyrosine phosphorylation of synthetic substrate was measured with a modified version of the method of Braun et al. [J. Biol. Chem. 259, 2051-2054 (1984)]. The PTK activity was measured at 20° C in a total volume of 64µl containing 50 mM Tris/HCl, ph 7.5, 20 mM Mg acetate, 5 mM NaF, 0.2 mM EDTA, 0.8 mM ethylenedioxy-bis(ethylenenitrilo)tetraacetic acid (EGTA), 30 µm Na₃VO₄, 1 mM dithiothreitol, 167 µm PGT (equivalent to 5 mM tyrosine residues) and 3 µg sample protein. The reaction was started by adding 50 µM [³²P]ATP having a specific activity of 0.75 Ci/mmol. After 12 min., the reaction was terminated by adding 25 µl of a solution containing 17.5 mM AtP and 70 mM EDTA. After terminating the reaction, aliquots of the mixture were placed on Whatmann 3MM paper, precipitated with TCA and washed thoroughly with 10% TCA and 100 mM PP₁. The paper discs were washed in ethanol/ether (1/1) and then in ether and dried in air. The incorporation of the label was determined quantitatively by means of liquid scintillation counting. The tyrosine kinase activity was calculated by subtracting the background phosphorylation of endogenous proteins, measured as described above in the absence of PGT subtrate. The PTK activity was expressed in pmol ATP/min/mg protein. The protein content was determined in accordance with Lowry et al. [J. Biol. Chem. 139, 265-275 (1951)].

### Preparation of phosphorylated PGT standard:

A phosphorylated PGT standard was prepared by incubating 200 µg of cytosol protein obtained from a breast carcinoma containing 1.6 mg PGT and 500 µM ATP in tyrosine kinase buffer (see above) for 22 hours at 4° C in a volume of 1.2 ml. The phosphorylation was terminated by adding 25 mM EDTA/EGTA (final concentration). The mixture was divided into small aliquots and stored at -70° C until the instant of use as a standard.

A phosphorylation reaction with [γ -³²P]ATP was carried out in parallel under identical conditions in order to determine quantitatively the phosphate incorporated in accordance with the TCA filter paper determination (see above). The incorporation was calculated as 3.5 mmol/mol tyrosyl residues.

### Determination of PTK activity according to the invention (dot-blot assay):

The PTK activity was determined in 30 µl of a mixture which contained 50 mM Tris/HCl, pH 7.5, 20 mM Mg acetate, 5 mM NaF, 0.2 mM EDTA, 0.8 mM EGTA, 1.0 mM dithiothreitol, 30 µm Na₃VO₄, 300 µM ATP, 50 µm PGT (equivalent to 1.8 mM Tyr residues) and 3 µg sample protein. The reaction was started by adding ATP. After incubating for 1 hour at 37° C, the phosphorylation reaction was terminated by adding 5 µl of a solution of 175 mM EDTA and 175 mM EGTA. The mixture was diluted with 140 µl water, after which 25 µl quantities of the mixture were placed on a PVDF membrane making use of a dot blot apparatus (Bio-Dot apparatus, Biorad, Richmond, CA, USA). The phosphotyrosine residues were then detected with the aid of the immunogold-silver staining procedure. Blocking of unoccupied membrane bonding sites, probing with primary antibody (1G2 antiphosphotyrosine, 2 µg/ml) and silver enhancement of the goldlabelled second antibody (goat antimouse IgG) were carried out in accordance with the instructions of the manufacturer of the respective reagents (Janssen, Belgium). The membrane was dried in air and the immunogold-silver signal was determined quantitatively by scanning the spots at 520 nm in a Beckman CDS 200 densitometer. The PTK activity was calculated from a phosphorylated PGT standard calibration curve. The activity was expressed in pmol ATP/min/mg protein.

### RESULTS:

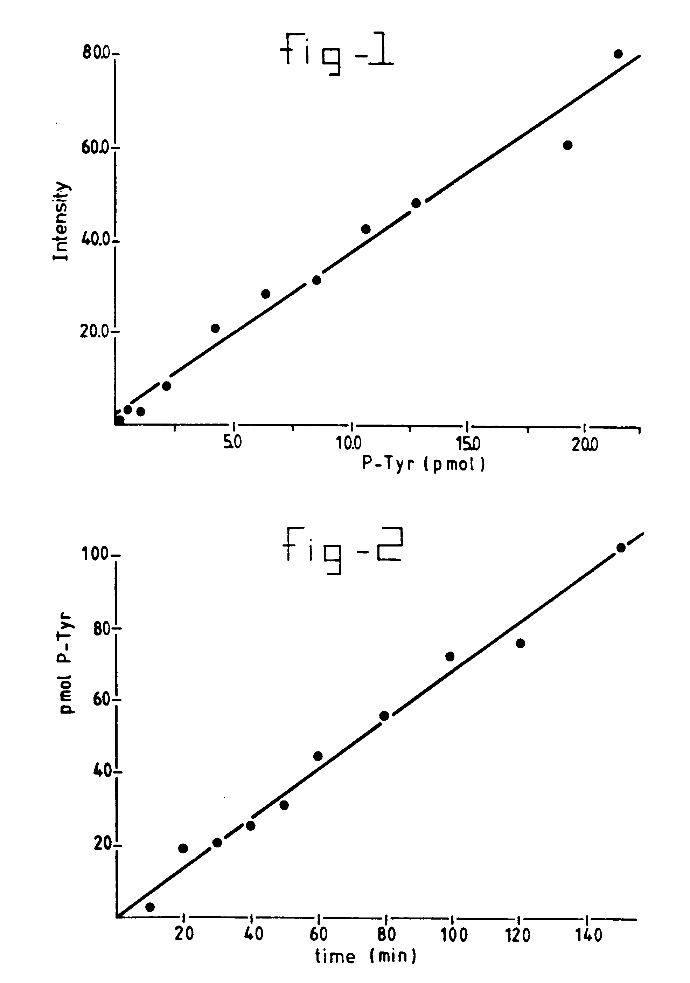

A phosphotyrosyl residue calibration curve was obtained by mixing unphosphorylated PGT and phosphorylated PGT standards in varying proportions. 5 µl quantities containing in total 5.7 µg (P)-PGT were placed on the PVDF membrane and these quantities correspond to those which were used in the PTK determination. Silver staining of the immunogold label and quantitative determination by means of densitometry yielded a linear response up to at least 20 pmol phosphotyrosine (Fig. 1). No background signal of unphosphorylated PGT was observed. The sensitivity of the method is such that a quantitative determination of less than 1.0 pmol phosphotyrosine is possible.

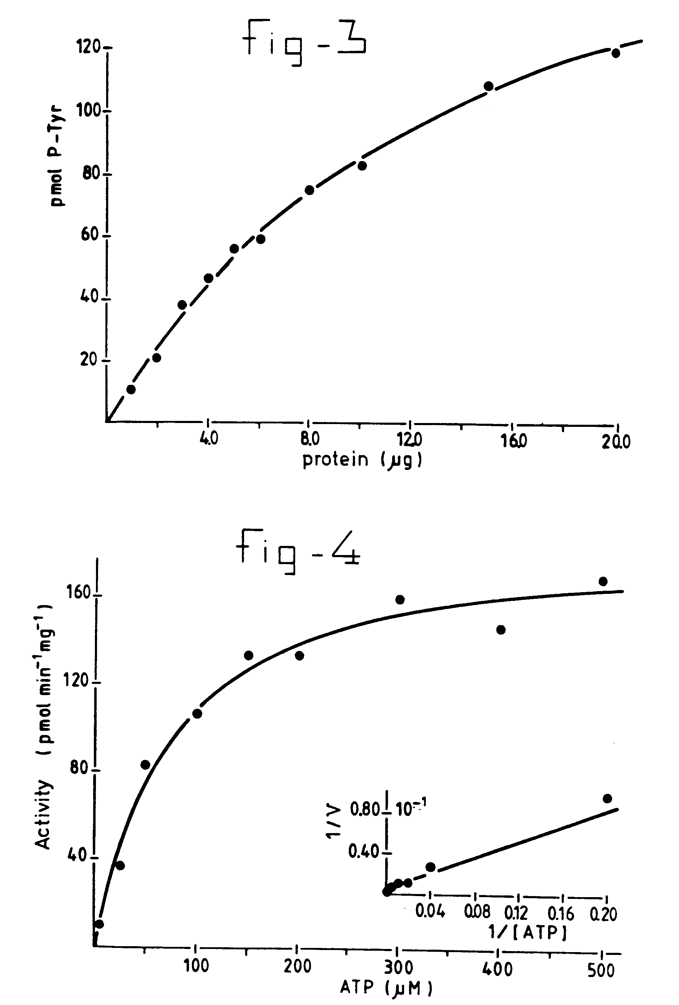

The calibration curves shown in Fig. 1 were used to determine quantitatively the PTK activity of the cytosol fraction of a breast carcinoma as a function of time and sample protein. Fig. 2 indicates that the reaction had a linear variation with time up to 2.5 hours. In control tests, the assays were carried out in the presence of EDTA/EGTA (25 mM each). Virtually no signal was obtained which indicated that the phosphorylation reaction is completely blocked by adding these reagents.

Variation in the amount of sample protein showed that the reaction had a linear variation with enzyme activity up to approximately 5 µg sample protein per assay (Fig. 3). With larger quantities of sample, the signal began to level off. In order to determine whether the signal originated specifically from the phosphorylation of added PGT or from the presence of endogenous proteins phosphorylated on tyrosine or from aspecific absorptions in the immunogold silver staining, parallel controls were carried out in the absence of PGT. A significant background staining became observable only with large quantities of sample protein (> 10 µg per assay), and this indicates that the contribution of both endogenous protein phosphorylation and of aspecific absorptions is quantitatively negligible in the signal.

Optimal substrate concentrations were determined on the basis of kinetic studies on the enzyme originating from breast carcinoma. To determine the Kₘ, PGT calibration curves were constructed in another manner: only the quantity of phosphorylated PGT standard was varied without adjusting the total quantity of (P)-PGT with unphosphorylated PGT. The calibration lines obtained in this process were essentially the same as those shown in Fig. 1, and from this it is evident that the bonding properties of the membrane for the substrate is not of decisive influence in these tests. The enzyme showed normal Michaelis-Menten kinetics both for ATP and for PGT (Figs. 4 and 5), with Kₘ ATP = 75 ± 13 µm and Kₘ PGT = 175 ± 39 µm (expressed in the concentration of tyrosyl residues). In the assay, 300 µM ATP and 1.8 mM Tyr (equivalent to 50 µM PGT) were therefore chosen as optimum concentrations.

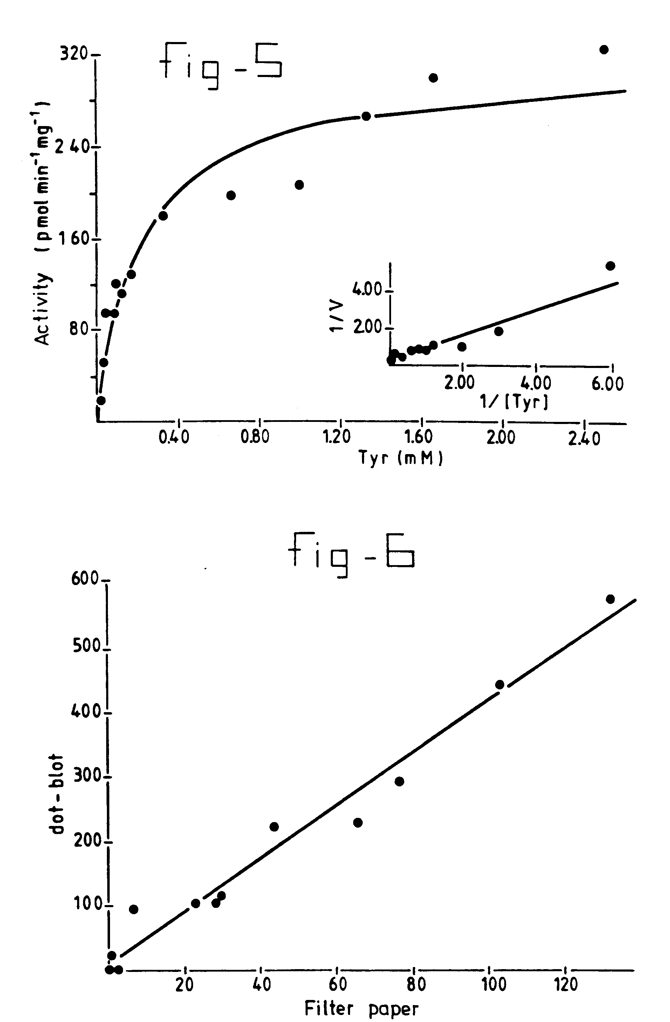

In the subsequent series of experiments, the conventional filter paper assay was compared with the new dot-blot assay according to the invention. Cytosol fractions were prepared from normal breast tissue (n = 2), benign breast tumours (n = 3) and malignant breast carcinomas (n = 5) and investigated for PTK activity. The comparison of the two determinations is shown in Fig. 6. A linear correlation was obtained with a correlation coefficient of 0.98. The activities determined according to the method of the invention were approximately 4 times as high as in the filter paper assay. This difference appears to be due mainly to the difference in incubation temperature and only to a small extent to differences in the substrate concentrations (results not shown). For practical reasons, the incubations with [³²P]ATP were carried at 20° C, while the incubation preceding the method according to the invention was carried out at 37° C. The lower detection limit of both assays was comparable. The sensitivity of the filter paper assay was, however, strongly dependent on the degree of background phosphorylation of endogenous protein (probably mainly on serine residues). As stated above, no background signal was observed in the determination according to the invention.

### EXAMPLE II

### Determination of the tyrosine phosphatase activity

200 µg of cytosol protein obtained from human granulocytes were incubated with 0.5 mg PGT and 0.5 mM ATP in tyrosine kinase buffer (see Example I) for 22 hours at 20° C in a volume of 1.2 ml. The phosphorylation was terminated by adding 110 µl 6 M TCA. After one hour at 4° C, the protein precipitate formed was centrifuged off (5 min., 12,000 x g). The pellet was washed a further three times with 0.6 M TCA. The last pellet was dissolved in 100 µl 1 N NaOH, after which the volume was made up to 2 ml with 50 mM Hepes, pH 7.0, containing 25 mM β-mercaptoethanol. A phosphorylation reaction with [³²P]ATP was carried out in parallel under identical conditions to determine the phosphate incorporated quantitatively in accordance with the TCA filter paper assay (see Example I). The incorporation was calculated at 0.8 mmol/mol tyrosyl residues.

The tyrosine phosphatase activity of a sample (for example, total cell extract of leukaemia cells) was determined in 30 µl of a mixture which contains 50 mM Hepes, pH 7.0, 25 mM β-mercaptoethanol and 15 µg phosphorylated PGT (equivalent to 20 pmol phosphotyrosyl residues). The reaction was started by adding that quantity of sample protein which is able to dephosphorylate not more than 3/4 of the available quantity of phosphotyrosyl residues in 30 min. at 37° C. After incubating for 30 min. at 37° C, the reaction was stopped by adding 5 µl of a solution of 0.25 mM Na₃VO₄. 5 µl quantities of the mixture were transferred to a PVDF membrane and the amount of phosphotyrosine residues was quantified as described in the PTK determination according to Example I.

## Claims

1. Method for determining the tyrosine-phosphorylating or phosphotyrosine-dephosphorylating activity of a biological material by bringing the material into contact with a polypeptide containing tyrosine or phosphotyrosine respectively and a radioactively unlabelled phosphate source or phosphate acceptor respectively and then assaying the phosphorylated polypeptide by using an antibody to polypeptide containing phosphotyrosine.

2. Method according to Claim 1, characterized in that the antibody is monospecific.

3. Method according to Claim 1 or 2, characterized in that the antibody is labelled.

4. Method according to Claim 1 or 2, characterized in that, to assay the phosphorylated polypeptide, a second, labelled antibody is also used.

5. Method according to Claim 3 or 4, characterized in that the labelled antibody is labelled with gold, whereby the signal of the goldlabelled antibody is optionally enhanced with silver.

6. Method according to Claim 1 or 2, characterized in that the polypeptide containing tyrosine or phosphotyrosine is labelled, preferably with an enzyme.

7. Method according to any one of Claims 1-6, characterized in that polypeptide containing tyrosine or phosphotyrosine is a synthetic polypeptide.

8. Method according to Claim 7, characterized in that the polypeptide also contains glutamic acid residues.

9. Method according to Claim 7 or 8, characterized in that the polypeptide has a random amino acid sequence.

10. Kit for determining the tyrosine-phosphorylating and/or phosphotyrosine-dephosphorylating activity of a biological material, which kit contains at least one tyrosine-containing polypeptide and/or a phosphotyrosine-containing polypeptide, a radioactively unlabelled phosphate source and/or phosphate acceptor, and an antibody to polypeptide containing phosphotyrosine.

11. Kit according to Claim 10, characterized in that it also contains a labelled antibody against the antibody to polypeptide containing phosphotyrosine.

12. Kit according to Claim 10 or 11, characterized in that the tyrosine- or phosphotyrosine-containing polypeptide is a synthetic polypeptide.

13. Kit according to Claim 12, characterized in that the polypeptide also contains glutamic acid residues and has a random amino acid sequence.

14. Kit according to one of Claims 10-13, characterized in that it also contains a carrier for immobilizing the polypeptide and/or the antibody, a medium suitable for carrying out the determination and other constituents such as an inhibitor, and an instruction for carrying out the method according to one of Claims 1-9.

## Patentansprüche

1. Verfahren zur Bestimmung der Tyrosin-Phosphorylierungs- oder Phosphotyrosin-Dephosphorylierungsaktivität eines biologischen Materials durch Kontaktieren des Materials mit einem Polypeptid, das Tyrosin bzw. Phosphotyrosin enthält, und einer radioaktiv unmarkierten Phosphatquelle bzw. einem Phosphat-Akzeptor und dann Testen des phosphorylierten Polypeptids unter Verwendung eines Antikörpers auf phosphotyrosinhaltiges Polypeptid.

2. Verfahren nach Anspruch 1, dadurch gekennzeichnet, daß der Antikörper monospezifisch ist.

3. Verfahren nach Anspruch 1 oder 2, dadurch gekennzeichnet, daß der Antikörper markiert ist.

4. Verfahren nach Anspruch 1 oder 2, dadurch gekennzeichnet, daß zum Testen des phosphorylierten Polypeptids ferner ein zweiter markierter Antikörper verwendet wird.

5. Verfahren nach Anspruch 3 oder 4, dadurch gekennzeichnet, daß der markierte Antikörper mit Gold markiert ist, wobei das Signal des goldmarkierten Antikörpers gegebenenfalls mit Silber verstärkt wird.

6. Verfahren nach Anspruch 1 oder 2, dadurch gekennzeichnet, daß das tyrosin- oder phosphotyrosinhaltige Polypeptid markiert ist, vorzugsweise mit einem Enzym.

7. Verfahren nach einem der Ansprüche 1-6, dadurch gekennzeichnet, daß das tyrosin- oder phosphotyrosinhaltige Polypeptid ein synthetisches Polypeptid ist.

8. Verfahren nach Anspruch 7, dadurch gekennzeichnet, daß das Polypeptid auch Glutaminsäurereste enthält.

9. Verfahren nach Anspruch 7 oder 8, dadurch gekennzeichnet, daß das Polypeptid eine statistische Aminosäuresequenz aufweist.

10. Kit zur Bestimmung der Tyrosin-phosphorylierenden und/oder Phosphotyrosin-dephosphorylierenden Aktivität eines biologischen Materials, wobei das Kit mindestens ein tyrosinhaltiges Polypeptid und/oder ein phosphotyrosinhaltiges Polypeptid, eine radioaktiv unmarkierte Phosphatquelle und/oder einen Phosphat-Akzeptor und einen Antikörper auf phosphotyrosinhaltiges Polypeptid enthält.

11. Kit nach Anspruch 10, dadurch gekennzeichnet, daß es auch einen markierten Antikörper gegen den Antikörper auf phosphotyrosinhaltiges Polypeptid enthält.

12. Kit nach Anspruch 10 oder 11, dadurch gekennzeichnet, daß das tyrosin- oder phosphotyrosinhaltige Polypeptid ein synthetisches Polypeptid ist.

13. Kit nach Anspruch 12, dadurch gekennzeichnet, daß das Polypeptid auch Glutaminsäurereste enthält und eine statistische Aminosäuresequenz aufweist.

14. Kit nach einem der Ansprüche 10-13, dadurch gekennzeichnet, daß es ferner einen Carrier zur Immobilisierung des Polypeptids und/oder Antikörpers, ein Medium, das zur Durchführung der Bestimmung geeignet ist, und weitere Bestandteile, wie einen Hemmstoff und eine Anleitung zur Durchführung des Verfahrens nach einem der Ansprüche 1-9, enthält.

## Revendications

1. Méthode pour la détermination de l'activité de phosphorylation de la tyrosine ou de déphosphorylation de la phosphotyrosine d'un matériel biologique par la mise en contact du matériel et d'un polypeptide contenant respectivement de la tyrosine ou de la phosphotyrosine et respectivement d'une source de phosphate non marqué radioactivement ou d'un accepteur de phosphate puis par la recherche du polypeptide phosphorylé en utilisant un anticorps dirigé contre le polypeptide contenant de la phosphotyrosine.

2. Méthode selon la revendication 1, caractérisée en ce que l'anticorps est monospécifique.

3. Méthode selon la revendication 1 ou 2, caractérisée en ce que l'anticorps est marqué.

4. Méthode selon la revendication 1 ou 2, caractérisée en ce que, pour rechercher le polypeptide phosphorylé, un second anticorps marqué est aussi utilisé.

5. Méthode selon la revendication 3 ou 4, caractérisée en ce que l'anticorps marqué est marqué avec de l'or, le signal de l'anticorps marqué à l'or étant optionnellement réhaussé avec de l'argent.

6. Méthode selon la revendication 1 ou 2, caractérisée en ce que le polypeptide contenant de la tyrosine ou de la phosphotyrosine est marqué, de préférence avec une enzyme.

7. Méthode selon l'une quelconque des revendications 1 à 6, caractérisée en ce que le polypeptide contenant de la tyrosine ou de la phosphotyrosine est un polypeptide de synthèse.

8. Méthode selon la revendication 7, caractérisée en ce que le polypeptide contient en outre des résidus d'acide glutamique.

9. Méthode selon la revendication 7 ou 8, caractérisée en ce que le polypeptide a une séquence d'acides aminés aléatoire.

10. Kit pour la détermination de l'activité de phosphorylation de la tyrosine et/ou de déphosphorylation de la phosphotyrosine d'un matériel biologique, lequel kit contient au moins un polypeptide contenant de la tyrosine et/ou un polypeptide contenant de la phosphotyrosine, une source de phosphate non marqué radioactivement et/ou un accepteur de phosphate, et un anticorps dirigé contre le polypeptide contenant de la phosphotyrosine.

11. Kit selon la revendication 10, caractérisé en ce qu'il contient en outre un anticorps marqué, anti-anticorps dirigé contre le polypeptide contenant de la phosphotyrosine.

12. Kit selon la revendication 10 ou 11, caractérisé en ce que le polypeptide contenant de la tyrosine ou de la phosphotyrosine est un polypeptide de synthèse.

13. Kit selon la revendication 12, caractérisé en ce que le polypeptide contient en outre des résidus d'acide glutamique et en ce qu'il a une séquence d'acides aminés aléatoire.

14. Kit selon une des revendications 10 à 13, caractérisé en ce qu'il contient en outre un support pour immobiliser le polypeptide et/ou l'anticorps, un milieu approprié pour la mise en oeuvre de la détermination et d'autres éléments tels qu'un inhibiteur et un enseignement, pour la mise en oeuvre de la méthode selon l'une des revendications 1 à 9.
